# EUROPEAN PATENT SPECIFICATION

(11) **EP 0 672 179 B1**
(45) Date of publication and mention of the grant of the patent: **08.07.1998**
(21) Application number: 93906753.4
(22) Date of filing: 25.03.1993
(51) Int. Cl.: C12Q 1/68

(54) **NUCLEIC ACID ANALYSIS**
NUCLEINSÄURE ANALYSE
ANALYSE D'ACIDE NUCLEIQUE

(30) Priority: 25.03.1992 GB 9206567
(43) Date of publication of application: 20.09.1995
(73) Proprietor: UNIVERSITY OF BRISTOL, Clifton Bristol BS8 1TH (GB)
(72) Inventor: BIDWELL, Jeffrey Lewis, Clevedon, Avon BS21 5HZ (GB)
(74) Representative: Nash, David Allan
(86) International application number: GB9300616
(87) International publication number: WO9319201

(56) References cited:
- EP-A- 0 256 630
- EP-A- 0 443 748
- WO-A-90/13668
- EUROPEAN JOURNAL OF IMMUNOGENETICS vol. 18, 1991, pages 147 - 153 N.A.P. WOOD ET AL. cited in the application

## Description

This invention relates to a method for examining single or multiple nucleotide polymorphisms (or mutations) in a nucleotide sequence in particular, but not exclusively, a nucleotide sequence contained within a sample of mammalian genomic DNA sequence.

Variations between individuals in the DNA sequence of genes coding for functional proteins account for many so-called "genetic diseases". For example, a single base pair substitution in the beta-globin gene leads to the genetic disease known as beta-thalassaemia. Similarly, these variations, particularly in the genes coding for functional Human Leucocyte antigens (HLA), explain many of the rejection problems encountered in human allogeneic transplant operations. Considerable effort is required to "HLA match" the donor to the recipient (patient).

Recently, a technique known as PCR (polymerase chain reaction) fingerprinting has been developed for human HLA-DR/Dw allotype matching (Bidwell JL and Hui KM 1990). As discussed in this paper, this application of the polymerase chain reaction technique permits the rapid matching between individuals of HLA-DR-Dw specifies. The technique exploits the formation of mismatched DNA hybrids (heteroduplexes) which are formed at the end of each PCR cycle between coamplified products of the second exons of expressed DRB genes (DRB1, DRB3, DRB4, and DRB5) and DRB pseudogenes (DRB2, DRB6, DRB7) (Also see Clay TM, Bidwell JL, Howard MR and Bradley BA 1991 and Wood NAP, Clay TM and Bidwell JL 1991). During the primer-annealing stage of each cycle of the PCR, a proportion of coding strands of each DRB locus may hybridise to noncoding strands of a different DRB locus and vice-versa. Resulting molecules contain double stranded regions where the nucleotide sequence is complementary, but regions of nucleotide sequence mismatch form single stranded loops along the length of the molecule. The number, size and position of the single stranded loops vary for each DR haplotype and each combination of haplotypes, in both DR-Dw homozygous and in heterozygous individuals. Because the molecular conformations of heteroduplexes vary, they may be separated from each other, and from the corresponding homoduplexes, by, for example, gel retardation analysis in nondenaturing polyacrylamide gels. In practice, haplotype-specific gel banding patterns (termed PCR fingerprints) are observed. Matching patterns of PCR fingerprints between two individuals indicate DR-Dw identity, whereas mismatched patterns indicate DR-Dw disparity: visual examination of ethidium bromide-stained polyacrylamide minigels thus constitutes a very simple test for DR-Dw compatibility.

PCR fingerprints observed in DR-Dw heterozygous individuals may be exactly reproduced by mixing DNAs from two corresponding DR-Dw homozygous typing cells (HTCs) prior to PCR (Bidwell and Hui 1990). However, in DR-Dw heterozygotes, additional bands may be observed which are not present in either of the patterns from the HTCs. These additional bands arise from heteroduplex formation in trans, that is, between PCR products from the two different haplotypes. This phenomenon is exploited in the DNA crossmatch test (Wood *et al* 1991, Clay *et al* 1991). Here, genomic DNAs from two different individuals are coamplified in the PCR. The resulting PCR fingerprint is compared with those obtained by separate PCRs for each individual. Matching PCR fingerprints from all three products indicates DR-Dw identity, whereas mismatch is indicated by different patterns and/or the appearance of new heteroduplexes formed in trans. This DNA crossmatching test may be successfully exploited to perform limited DR-Dw "typing": for example, DNA crossmatches with DR7-DQ9 and DR9-DQ9 HTCs may be used to discriminate between these alleles where conventional DNA-RFLP typing fails.

Theoretically, the number of conformationally distinct heteroduplexes generated from a DR-Dw homozygous or heterozygous individual with a total of *n* different alleles at coamplifiable loci, is *n*² - *n* (Bidwell and Hui, 1990). In practice, particularly in DR-Dw heterozygotes, not all of the predicted conformationally distinct heteroduplexes are observed. A likely reason for this incomplete resolution is that a proportion of heteroduplexes comigrate and are thus superimposed in gel retardation analysis. In addition, some heteroduplexes may form less avidly or be less stable than others. In general, however, there are more than sufficient discriminatory heteroduplexes to permit matching, but in a few cases (for example, discrimination between certain DR4 alleles in DR4-homozygous individuals), PCR "spiking" may be necessary. PCR spiking is the inclusion or "spiking" of PCRs with DNA from a DR-Dw disparate HTC, for example a DR8 HTC. Spiking PCRs in this manner with third party DNA generates new heteroduplexes which enhance discrimination between otherwise similar PCR fingerprints (Clay *et al* 1991 and Bidwell JL 1992). DNA from a homozygous DR8 cell line has been successfully used as a "spiking" reagent since it generates a defined but limited number of new heteroduplexes.

Although PCR fingerprinting works well for HLA-DR/Dw matching, extension of the principle to matching HLA-DP and DQ (other loci in the human HLA region coding for alloantigenic functional proteins) is not directly achievable because there are insufficient amplifiable loci. Moreover, the PCR fingerprinting technique is unsuitable as a generalised method for examining single or low numbers of dispersed nucleotide polymorphisms, such as those which result in genetic diseases.

According to a first aspect of the present invention, there is provided a method for examining polymorphisms in a nucleotide sequence of a first sample of nucleic acid comprising the steps of:
(a) forming a population of nucleic acid fragments bearing the said nucleotide sequence;
(b) combining the population of nucleic acid fragments with a population of a synthetic nucleotide construct which construct is capable of forming duplexes with the nucleic acid fragments, the sequence of the construct being such that duplexes of different molecular conformation are formed between the construct and the nucleic acid fragments dependent upon the presence of a polymorphism at a known variable nucleotide or sequence of nucleotides within the sequence of the sample under examination;
(c) permitting duplex formation within the combined populatidns; and
(d) separating the duplexes formed in step (c).

The synthetic construct contains one or more deliberate nucleotide substitution(s) and/or deletion(s) and/or insertion(s) which is/are (i) opposite to a known variable nucleotide or sequence of nucleotides within the sequence under examination and/or (ii) contiguous with a nucleotide which is opposite to a known variable nucleotide or sequence of nucleotides within the sequence under examination.

By the term "opposite", we mean that, in the resultant duplex between the nucleic acid fragment strand and the synthetic construct strand, the deliberate nucleotide substitution(s) and/or deletion(s) and/or insertion(s) is/are in the position(s) in the synthetic construct strand corresponding to the variable nucleotide(s) under examination. The "contiguous" portion of the synthetic construct strand may be a single nucleotide in contact with the nucleotide opposite the variable nucleotide(s) or may be a nucleotide sequence, normally no more than 20 bases in length, in which one of the end nucleotides is in contact with the nucleotide opposite the variable nucleotide(s).

For the avoidance of doubt, it should be understood that, in the present invention, the nucleotide substitution(s) and/or deletion(s) and/or insertion(s) contained in the sequence of the construct are made either (i) relative to the wild type of the nucleotide sequence under examination so that, in the duplexes formed between the nucleic acid fragments and the construct, there is created a deliberate or controlled mismatch when the nucleotide sequence contains a mutation from the wild type or (ii) relative to a mutant of the nucleotide sequence under examination so that, in the duplexes formed between the nucleic acid fragments and the construct, there is created a deliberate or controlled mismatch when the nucleotide sequence is either wild type or another (different) mutant.

Preferably, the nucleotide sequence of the synthetic construct at least contains deliberate nucleotide substitution(s) and/or deletion(s) and/or insertion(s) contiguous with the nucleotide which is opposite the known variable nucleotide or sequence of nucleotides. It may also contain deliberate nucleotide substitution(s) and/or deletion(s) and/or insertion(s) opposite the known variable nucleotide or sequence of nucleotides.

Preferably, the synthetic construct includes nucleotide substitution(s) and deletion(s) both opposite and contiguous with the mutation. In practice, it has been found that a sequence of between one and ten substitutions/deletions adjacent the position of the polymorphism in the nucleotide sequence under investigation is satisfactory for the purposes of this invention.

In order simultaneously to identify more than one polymorphism in a given nucleotide sequence in a single or reduced number of experiments, the sequence of the construct is such that duplexes of different molecular conformation are formed between the construct and the nucleic acid fragments dependent upon the presence or absence of polymorphisms at two or more known variable nucleotides and/or sequences of nucleotides within the sequence of the sample under examination; it is therefore preferred that the synthetic construct includes deliberate nucleotide substitution(s) and/or deletion(s) and/or insertion(s) corresponding to at least two known polymorphisms in the nucleotide sequence, and preferably corresponding to all of the known polymorphisms in the nucleotide sequence.

The extent of deliberate nucleotide substitution(s) and/or deletion(s) and/or insertion(s) should not be so great that duplex formation between the nucleic acid fragments and the construct is not possible.

The construct may also include a deliberate deletion or a series of deletions, at a position away from polymorphic sites in the nucleotide sequence under investigation (this is in addition to any deletion adjacent to a mutation site) ; this has the effect of permitting better electrophoretic separation of heteroduplexes from homoduplexes.

The results of the separation conducted in step (d) of the method of this invention may be compared with the results of one or more similar separations conducted on duplexes formed using steps (b) to (d) above with the same synthetic nucleotide construct and a population of nucleic acid fragments bearing a nucleotide sequence corresponding to the said nucleotide sequence under examination, but taken from one or more different samples of DNA or RNA. This enables polymorphisms in any of the nucleic acid samples to be detected and compared.

Typically, the nucleic acid under examination will be DNA, normally mammalian (such as human) genomic DNA. In its broadest aspect, however, the nucleic acid may be DNA or RNA from any source, genomic or otherwise, for example, a virus, bacterium, plant or animal source.

The synthetic nucleotide construct will normally be a synthetic DNA, because this will form the most stable duplexes with DNA fragments containing the nucleotide sequence under investigation.

Where the nucleic acid under examination is mammalian genomic DNA, a sample of the DNA is obtained from an individual or other object whose genotype for a specific characteristic it is wished to study. (The term "individual" is intended to include a foetus.) DNA can be extracted from all nucleated cells. Typically, the DNA is obtained from peripheral blood cells for convenience. Foetal DNA can be obtained from placental cells or amniotic fluid. Other sources of DNA include hair follicles, mummified bodies, etc.

The DNA may be isolated by any appropriate method, for example by the rapid salting out method described by Miller *et al* (Miller, S., Dykes, D. and Polesky, H. (1988) "A simple salting out procedure for extracting DNA from human nucleated cells"; *Nucl. Acids Res.* 16:1215). Alternatively, the DNA may be isolated as cDNA from mRNA by reverse transcription.

The nucleotide sequence contains the polymorphism under examination; thus, the sequence will include at least that part of the relevant gene containing the polymorphism. This will normally be a part of the gene ultimately coding for a region of the functional protein (i.e. exon), but may alternatively or additionally be an intervening part of the gene which is not responsible for coding the functional protein (e.g. an intron) but which nevertheless affects transcription and translation or is known to be in genetic linkage with specific polymorphisms in coding sequences of the gene. Since two or more loci coding for different forms of the same protein - or representing pseudogenes - may be present (as for the DRB genes of the HLA-DR loci) so there may be other forms of the nucleotide sequence under investigation and these other forms of the nucleotide sequence may be formed into a population of nucleic acid fragments which can participate in the duplex formation reaction of step (c) - for example see Bidwell, JL and Hui, KM (1990).

The population of nucleic acid fragments bearing the nucleotide sequence under examination is formed from the first sample of nucleic acid by a method which preserves the relevant sequence information. It is presently preferred that the method used is one which directly amplifies the nucleotide sequence in the sample of DNA or RNA (which may for example be isolated directly from the organism under investigation or may be cDNA derived from mRNA by reverse transcription) to result in a population of nucleic acid fragments bearing the nucleotide sequence. Depending upon the method of amplification employed, the resulting nucleic acid fragments may be RNA or DNA. However, they are preferably DNA, formed from a DNA sample using an enzymatic amplification technique such as the polymerase chain reaction (PCR). The technique of "run-off" transcription (Green, MR, Maniatis, T, Melton, DA (1983) Human beta-globin pre-mRNA synthesised in vitro is accurately spliced in Xenopus oocyte nuclei. Cell 32:681-694) may be used to prepare a population of RNA fragments from a DNA sample.

The preferred method for forming from a DNA source a population of DNA fragments bearing the nucleotide sequence is the polymerase chain reaction (PCR). In this procedure, two oligonucleotide primers are added to the DNA source for annealing to complementary sequences at either end of the nucleotide sequence, a heat-stable DNA polymerase, such as Taq polymerase, dATP, dCTP, dGTP and dTTP and appropriate biological buffers and cofactors. The DNA is denatured, the oligonucleotide primers anneal to their complementary sequences with the 3' ends pointing towards each other and the DNA polymerase results in extension of the annealed primers and amplification of the segment of DNA defined by the 5' ends of the primers.

Where the synthetic construct is a DNA construct, the population of the synthetic DNA construct may be formed using the PCR, preferably employing the same primers as used for amplification of the nucleotide sequence under consideration although different primers could be used so long as the resulting amplified construct is capable of forming the necessary duplexes with the nucleic acid fragments.

In the PCR, the cycle of DNA denaturation, primer annealing and synthesis of the DNA segment defined by the 5' ends of the primers is repeated as many times as is necessary to amplify the nucleotide sequence under consideration (or the construct) until sufficient is available for step (c) of the present method. Amplification may proceed for from 20 to 40 cycles, for example from 25 to 35 cycles. Amplification of the nucleotide sequence under investigation and the construct may be conducted simultaneously in a mixed PCR or in separate PCRs.

Any appropriate oligonucleotide primers may be employed. The primers should be suitable for amplification of specific locus under investigation.

The primers may be labelled for facilitating the comparison between the results of the separation in step (iii) of the present method and the separation conducted on the duplexes formed in the second reaction on the second sample of DNA. The primers can be labelled with a directly detectable tag, for example a radionuclide such as ³²P or ³⁵S, a fluorescent compound such as fluorescein, an enzyme such as a horseradish peroxidase or alkaline phosphatase, with biotin or digoxigenin. The two primers may have the same or different labels.

Before formation of a population of the construct (for example by using the PCR), the construct must first be synthesised. Typically, the construct is synthesised from multiple synthetic oligonucleotides, for example. Once synthesised, it may be cloned in a suitable host, for instance an M13 phage.

The amplified nucleic acid fragments and the amplified construct obtained from mixed PCR or separate PCRs are heated (mixed PCR) or mixed and heated (separate PCRs) to permit separation of the amplified product into single stranded nucleic acid which is then allowed to re-anneal under suitable annealing conditions to form the duplexes. Duplexes will be formed between the coding and non-coding strands present in the reaction vessel and, in particular, a proportion of the duplexes so formed will be mismatched hybrids between sense and anti-sense strands of partially homologous sequences ("heteroduplexes"), formed either in cis or trans or in both cis and trans.

The heteroduplexes formed are separated according to their molecular conformation which affects their apparent, but not actual, molecular weight. This may be achieved by, for example, electrophoresis. The separation is typically effected on a gel which does not fully denature the nucleic acid, such as a nondenaturing polyacrylamide gel. Electrophoresis is conducted under conditions which effect a desired degree of resolution of the duplexes. A degree of resolution that separates duplexes that differ in "apparent size" - resulting from their different molecular conformations - by as little as about 10 bp is usually sufficient. The heteroduplexes are "shifted" away from the homoduplexes in the separation by introducing one or more deliberate deletions in the construct as described above; this has the effect of retarding the mobilities of all heteroduplexes within the gel. Size markers may also be run on the gel to permit estimation of the apparent size of duplexes.

The distribution, i.e. the resolution pattern, of the heteroduplexes will be allele-specific. This resolution pattern or PCR fingerprint can next be visualised. Where a PCR primer has been labelled, this label may be revealed. A substrate carrying the separated labelled duplexes is contacted with a reagent which detects the presence of the label. Where the PCR primers were not labelled, the substrate bearing the PCR fingerprint may be contacted with, for example, ethidium bromide and the nucleic acid fragments visualised under ultraviolet light; alternatively, the heteroduplexes may be visualised with silver staining.

The relative molecular conformation of the duplexes is thus determined, in this embodiment, as a pattern of bands representing the duplexes which migrate different distances from the origin on the gel, dependent on their relative molecular conformation. This is compared with the relative molecular conformation of duplexes resulting from a similar exercise (using the same construct) conducted on a nucleotide sequence of a second sample of nucleic acid. In this way, correspondence between the nucleic acid samples may be obtained. It may be ascertained whether the relative molecular conformation of the duplexes obtained, and therefore whether the nucleotide sequences of the two samples are the same or not.

The relative molecular conformation in respect of the second sample of the nucleic acid (typically DNA) may be obtained using the same conditions as are employed to obtain the relative molecular conformation in respect of the first nucleic acid sample. Typically the same primers are used. PCR amplification need not necessarily be for the same number of cycles or under identical reaction conditions, though. Similarly, separation of the resulting duplexes need not be carried out in an identical fashion provided it is possible to assess the relative correspondence of the molecular conformation of the duplexes resulting from amplification of each sample.

The second sample of nucleic acid may be analysed according to the present method simultaneously with or at a different time to analysis of the first nucleic cid sample. Indeed, a multiplicity of samples may be analysed. Typically the or each sample is a selected sample. Samples from selected individuals can be analysed. The relative molecular conformation determined for each sample may be held in a computer.

A computer database may therefore be generated containing the relative molecular conformation distribution patterns for different samples.

The PCR fingerprint using the method of the invention may be compared with another PCR fingerprint to determine whether the individuals, whose DNA has been tested to obtain the two fingerprints, match in respect of the particular nucleotide sequence under investigation. The present method can therefore be applied to DNA samples from two or more individuals. Alternatively, a PCR fingerprint may be compared with a standard or reference fingerprint previously obtained. Correspondence between fingerprints can therefore be determined.

The present method can be used for example to determine whether a donor of a transplant or transfusion and a recipient or proposed recipient of the transplant or transfusion have matching allotypes. PCR fingerprints from the donor and the recipient or proposed recipient can be compared. The transplant may be a tissue transplant such as a heart, lung, liver or kidney transplant or a bone marrow transplant. The transfusion may be a blood transfusion. Matching of living related or unrelated donors for allogeneic transplantation may therefore be achieved.

In particular, the method can be used in matching individuals in respect of one or more of the functional proteins expressed by the HLA (human leucocyte antigen) class I and II genes, for example the Class II DPB, DQB, DQA and DRB genes which all exhibit polymorphism. This may be used for "typing" individuals and "matching" a patient to a donor in a transplant operation. Where the DPB gene is under investigation, it has been found effective to analyse the second exon of the gene which is the known polymorphic sequence of the gene. A DNA construct for use in the method of the invention for analysis of the DPB1 second exon should preferably have a nucleotide sequence substantially corresponding to the invariable regions of the gene and nucleotide substitutions at the known variable nucleotides, for instance at one or more of positions 56, 58, 59, 60, 65, 66, 138, 141, 198, 199, 201, 205, 227, 239, 260, 262, 285, 288, 290, 293 starting from the 3'- end of primer UG19 (a known primer used to prime DNA polymerisation of the second exon of DPB1). In addition, the construct contains at least one deletion when compared with the gene sequence under consideration, for instance a 5 base pair deletion at position 235 starting from the 3'- end of primer UG19 (SEQ ID NO: 6). This is in order more effectively to separate the heteroduplexes from the homoduplexes.

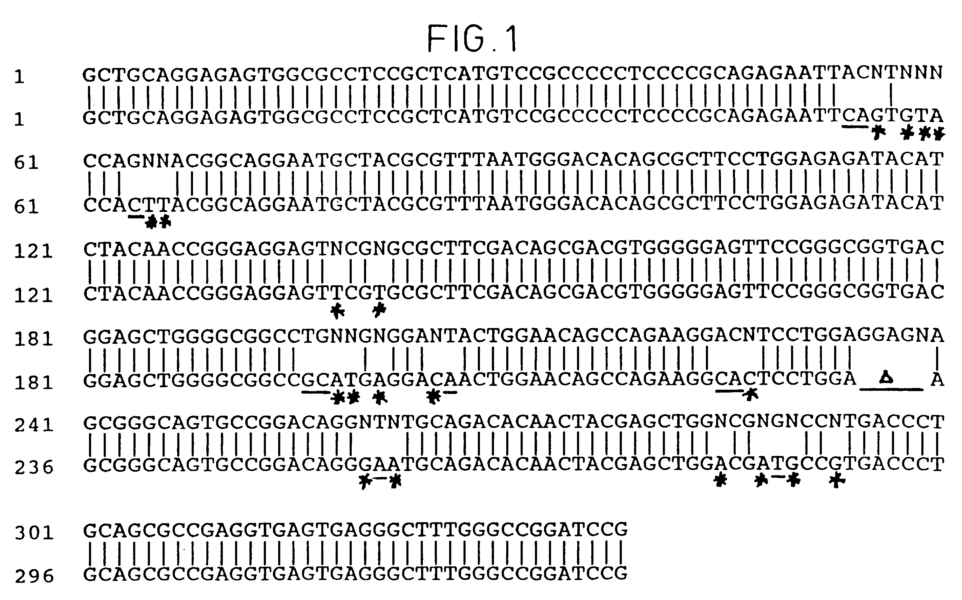

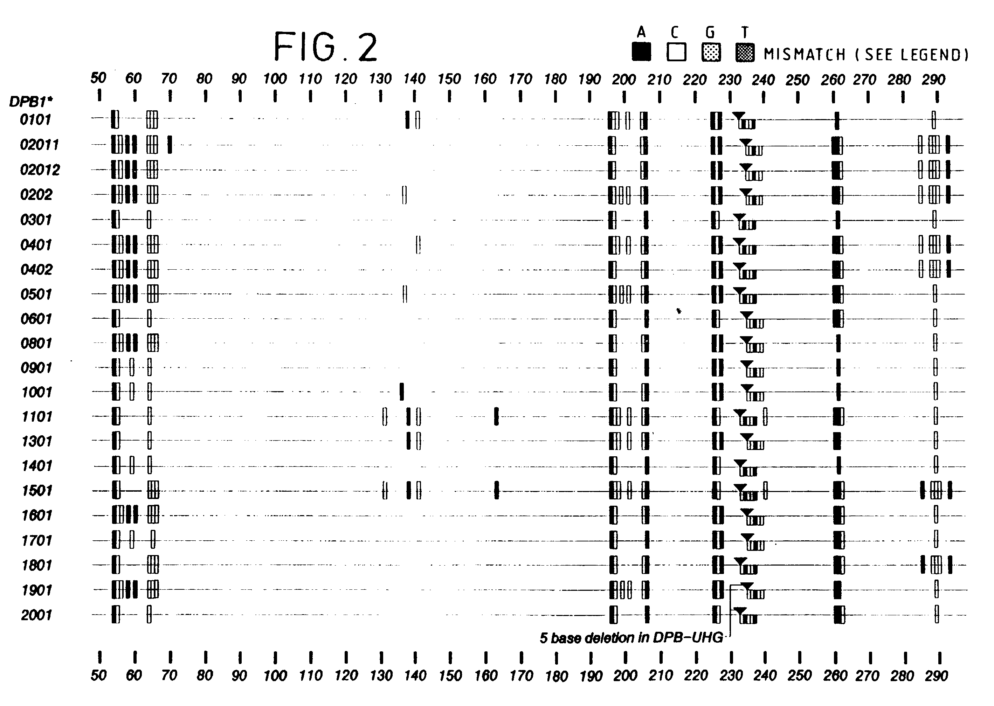

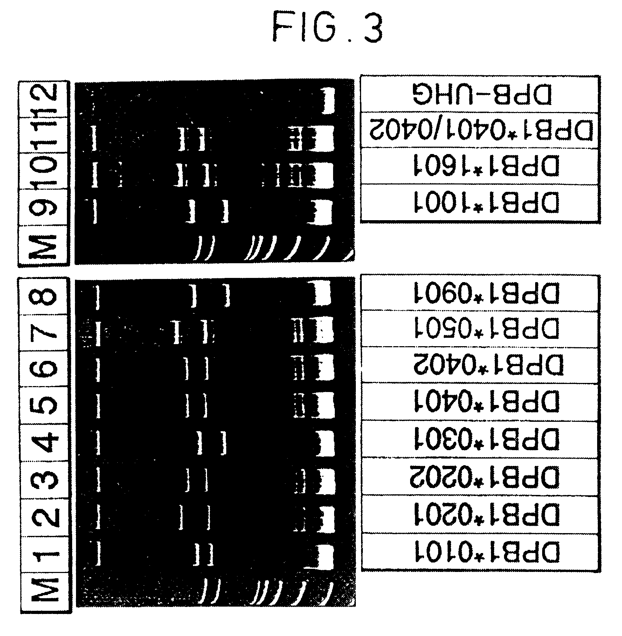

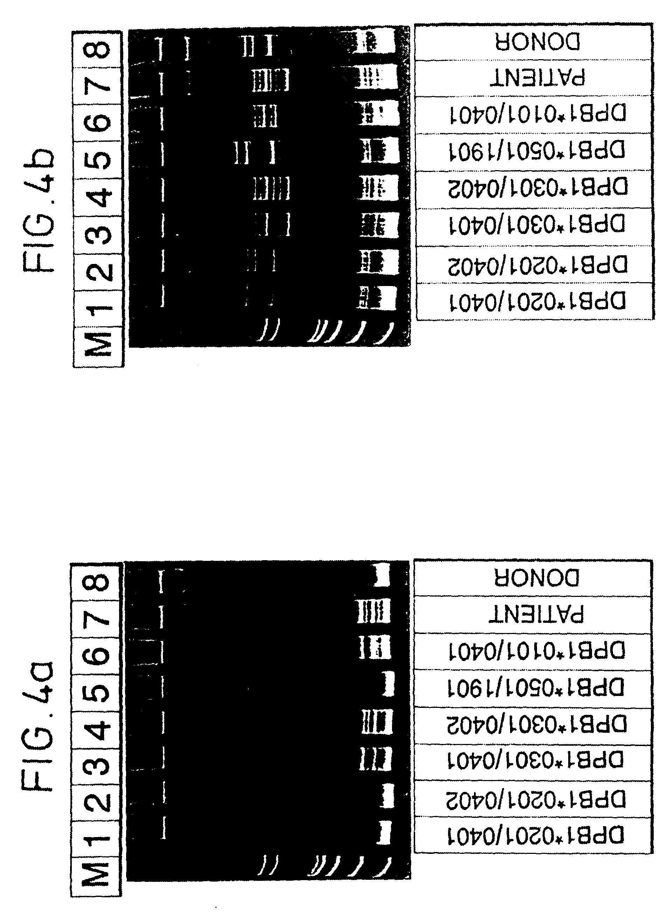

Thus, whilst extension of the heteroduplex principle as described by Bidwell and Hui (1990), supra to HLA-DP matching is not directly possible because for DPB1-specific PCR primers there is generally only a single PCR product, nevertheless it is possible with the method of the present invention to generate PCR fingerprints for the DPB1 locus by permitting heteroduplex formation between the population of DNA fragments bearing the nucleotide sequence under examination with a synthetic molecular construct (such as that described above) which contains at least one deliberate nucleotide substitution, deletion or insertion, designed to be opposite and/or contiguous with a hypervariable nucleotide within, for this example, polymorphic second exons of the target DPB1 genes. These constructs may be used following PCR reactions in order to produce heteroduplexes specific for individual DPB1 alleles, and thus to allow rapid matching. We have constructed and cloned a construct (see Figure 1) specific for the DPB1 locus which discriminates between the 21 specificities DPB1*0101 to DPB1*2001 (Fig 2). In practise, DPB1 second exon sequences are amplified from genomic DNA in the normal manner. In a separate PCR and using the same primers, the synthetic "DPB construct", or universal heteroduplex generator (UHG), is selectively amplified from an M13mp18 phage clone. Next, aliquots from the two separate reactions are mixed together and subjected to 2-3 further cycles of PCR, in order to allow heteroduplex formation. Finally, PCR fingerprints are examined in the normal way following polyacrylamide gel electrophoresis. Fig 3 shows the results obtained for a selection of DPB1 HTCs. In DPB1 heterozygotes, some naturally occurring heteroduplexes formed in trans might be expected, but in practice not all combinations of alleles produce heteroduplexes without use of the method of the present invention employing a DPB construct (Fig 4a). However, conducting the method with an amplified DPB construct yields highly discriminatory PCR fingerprints of heteroduplexes (Fig 4b), analogous to the DR-Dw system. Newly described alleles (for example, DPB1*2101-3601) may be catered for by alteration to one or more nucleotides within an existing construct. It is envisaged that this method will be particularly appropriate to the DQA1 and DQB1 loci, and in conjunction with group-specific PCR-SSP, to problematic areas in DRB1, for example DR4 subtype matching.

The present method can also be used in determining whether an individual is susceptible to or has a disease associated with a particular nucleotide sequence which codes for a functional protein. Such diseases are reviewed in Immunol. Rev. 70, 1-218, 1983.

In a method in accordance with the present invention for the diagnosis of a genetic disease, a suitable synthetic construct (usually a DNA construct) is employed which is capable of forming duplexes with a nucleotide sequence of (a) a translated or non-translated exon region, or (b) an intron region which affects expression or mRNA slicing, the nucleotide sequence containing a mutation responsible for a genetic disease, the construct containing one or more deliberate nucleotide substitution(s) and/or deletion(s) and/or insertion(s) which is/are (i) opposite to a known variable nucleotide or sequence of nucleotides within the sequence under examination and/or (ii) contiguous with a nucleotide which is opposite to a known variable nucleotide or sequence of nucleotides within the sequence under examination. DNA is then isolated from the individual under diagnosis, for example from whole blood obtained according to standard protocols or from dried blood spots or from any other suitable source of DNA. Populations of the chosen synthetic construct and the nucleotide sequence are formed, for example using the polymerase chain reaction and these populations are then combined under suitable conditions to permit duplex formation within the combined populations. The duplexes formed are then separated, for example using gel electrophoresis, and the duplexes visualised, for example by staining. The resulting profile is then compared with a standard profile representing the results obtained using the same synthetic construct either with a DNA sample known to contain the mutation under examination or a DNA sample known not to contain the mutation under examination. This enables the user of the method readily to identify whether the individual under diagnosis carries the mutation responsible for the genetic disease.

The present invention is particularly suitable where the relevant gene is multiallelic but is not duplicated within the genome and therefore DNA heteroduplex formation following the polymerase chain reaction (PCR) is possible only in 'trans' and therefore only in heterozygotes for a given gene. In accordance with the present invention, DNA heteroduplexes can be generated by hybridising PCR products with synthetic DNA molecules. These are PCR-amplifiable DNA sequences that mimic a DNA sequence but differ from it as a result of controlled nucleotide substitution(s), deletion(s) or insertion(s) at sites continuous to known mutation sites within the DNA. The construct and DNA sequences are amplified with the same PCR primers, and hybridised together post-PCR by heating and slow cooling, resulting in DNA heteroduplexes having different conformational forms, and thus different electrophoretic mobilities, unique for individual alleles of a gene (Bidwell et al, 1992).

The use of the present method is particularly suitable in, for example, phenylketonuria genotyping. Phenylketonuria (PKU) is probably the most common disorder of amino acid metabolism and results from mutations at the phenylalanine hydroxylase (PAH) gene. The gene consists of 13 exons and encodes a MRNA of 2.8 kilobases (Kwok et al, 1985; DiLella et al, 1986). To date, more than 50 mutations have been described at the PAH gene but there are wide variations in the frequency distribution of individual mutations between different populations (Scriver et al, 1992). In the south west of England, three mutations in exon 12, namely IVS12nt1, R408W and Y414C, account for just over 40% of PKU chromosomes (Tyfield et al, 1991). In vitro expression analyses reveal 100% loss of PAH activity associated with IVS12nt1 and R408W mutations, and 50% reduction in activity for the Y414C mutation (Okano et al, 1991a, b). Within this population, other mutations found in the PAH gene are in exon 3 (I65T) and intron 10 (IVS10nt546), resulting in 74% and 100% loss of PAH activity respectively (John et al, 1992; Dworniczak et al, 1991).

A significant proportion of early treated young adult PKU sufferers show clinical neurological deterioration (Thompson et al, 1991; Lou et al, 1992), and thus it is important to research the possible link between specific PAH gene mutations and long term prognosis. Because of the large number of disease-associated mutations at this locus it is desirable to have available a mutation detection system which is rapid, safe and reliable, and is able to screen samples for several mutations at one time.

Several techniques of mutation detection have been described, including those that depend on conformational polymorphisms in amplified single stranded DNA (SSCP) (Orita et al,1989), chemical cleavage of mismatched DNA sequences in amplified DNA (Cotton et al, 1988), and those that depend on differences in electrophoretic melting properties between amplified heteroduplexes and homoduplexes in denaturing or thermal gradient gels (DGGE and TGGE) (Fisher and Lerman, 1983). However, none of these techniques is without some limitation regarding safety, reliability or universal adaptability.

In a method in accordance with the present invention for the diagnosis of a PKU, it has been found to be effective in many cases to analyse exon 12 of the PAH gene which contains at least five known mutation sites leading to full or partial inactivation of the PAH gene product. Thus the DNA construct should preferably have a nucleotide sequence substantially corresponding to an invariable region or regions of the nucleotide sequence of exon 12 of the gene, with at least one nucleotide substitution, deletion or insertion at or adjacent the known variable nucleotides. Thus, for instance, the DNA construct may have such a deletion, substitution or insertion at or adjacent to one or more of the following four positions, defined by reference to the specific codons of the PAH exon 12 wild type gene:
the 1st nucleotide of codon 408;
the 2nd nucleotide of codon 413;
the 2nd nucleotide of codon 414; and
the 1st nucleotide of codon 418;
and may also have such a deletion, substitution or insertion at or adjacent the position corresponding to the 1st nucleotide of the PAH intron 12 wild type gene. Preferably, deletions and substitutions are employed.

The invention also provides a test kit, which kit comprises:
(a) two oligonucleotide primers suitable for use in PCR and capable of annealing to complementary sequences at respective ends of a nucleotide sequence to be examined;
(b) a synthetic nucleotide (preferably DNA) construct which construct is capable of forming duplexes with the nucleotide sequence under consideration, the sequence of the construct being such that duplexes of different molecular conformation are formed between the construct and the nucleotide sequence under examination dependent upon the presence of a polymorphism at a known variable nucleotide or sequence of nucleotides within the sequence under examination; and
(c) a control DNA and/or control PCR amplification product.

The synthetic nucleotide construct contains one or more deliberate nucleotide substitution(s) and/or deletion(s) and/or insertion(s) which is/are (i) opposite to a known variable nucleotide or sequence of nucleotides within the sequence under examination and/or (ii) contiguous with a nucleotide which is opposite to a known variable nucleotide or sequence of nucleotides within the sequence under examination;

The primers may be labelled as above. The control PCR amplification product may also be labelled. The kit may further comprise one or more of the following:
- a heat-stable DNA polymerase;
- DATP, DCTP, DGTP and DTTP;
- appropriate biological buffers and cofactors;
- a database comprising the relative molecular conformation and gel mobilities of DNA fragments generated by PCR amplification and heteroduplex formation between selected DNA samples and the construct.

For a better understanding of the present invention, and to show how the same may be carried into effect, reference will now be made to the accompanying drawings, in which:
Figure 1 shows a "consensus" sequence for the second exon of the DPB-1 gene and a 5'- flanking (intron) region and the corresponding sequence of a synthetic DNA construct for use in a method in accordance with this invention. The sequence of the synthetic DNA construct is SEQ ID NO: 1 in the attached sequence listing.
Figure 2 shows a PCR fingerprint for HLA-DPB1 alleles produced in accordance with the present invention using a construct having the sequence shown in Figure 1;
Figure 3 shows the PCR fingerprints for HLA-DPB1 homozygous typing cells using the method of the present invention and the construct having the sequence shown in Figure 1;
Figure 4a shows the PCR fingerprints of HLA-DPB1 heterozygotes, without use of a construct;
Figure 4b shows corresponding PCR fingerprints to those shown in Figure 4a, but using the construct of Figure 1 in the PCR fingerprinting method;
Figure 5 shows the nucleotide sequence of a portion of the normal human PAH gene, including exon 12 and part of the 5' and 3' flanking introns, as well as 5 genotypes including known PKU mutations and a DNA construct for use in identifying those mutations;
Figures 6a, 6b, 7 and 8 show the stained gels for a series of experiments on the of PAH gene exon 12; and
Figures 9(a), 9(b) and (c) model the homoduplexed and synthetic construct-heteroduplexed nucleotides between codons 407 and 410 of exon 12 of the PAH gene using in the model the construct sequence which identifies the R408W mutation.

### EXAMPLE 1

A DNA construct specific for the DPB1 locus was synthesised from multiple synthetic oligonucleotide "longmers", each of about 100 nucleotides in length, using techniques known in the art. In this example, four longmers were employed having the sequences identified SEQ ID NO: 2, SEQ ID NO: 3, SEQ ID NO: 4 and SEQ ID NO: 5. The DNA sequence of the construct was chosen to overlap the start of the second exon of the DPB1 gene, the nucleotide sequence of which is known from Marsh, SGE and Bodmer, JG. (1991) "HLA Class II nucleotide sequences, 1991"; *Tissue Antigens,* 37: 181-189, and contained deliberate nucleotide substitutions and a deletion designed to be contiguous with known hypervariable nucleotides within the sequence of the second exon of the DPB1 gene. The construct was 338 base pairs in length. The construct sequence included 29 mismatches and 5 unmatched bases between the construct and a "consensus" sequence for the second exon and 3' flanking intron of the DPB1 gene, the consensus sequence being determined by using a computer alignment program, MicroGenie (Version 7.01), Beckman Instruments. The construct was cloned in an M13mp18 phage.

The DPB1 "consensus" sequence is the top line in Figure 1. This "consensus" sequence was determined using a computer alignment program and variable bases are identified by the letter N. This means that these particular bases in the DPB1 second exon vary between individuals and give rise to the various allelic forms of the gene. For simplicity, the 5'-3' coding strand of the consensus sequence only is shown.

The bottom line of Figure 1 (also SEQ ID NO: 1) is a DNA construct designed to contain deliberate nucleotide substitutions and deletions contiguous with the known variable nucleotides within the nucleotide sequence (here the second exon of the DPB1 gene) under examination. Again, the 5'-3' coding stand is shown for simplicity.

As will be noted, the construct is designed to have an identical sequence with the great majority of the non-variable nucleotides of the DPB1 consensus sequence, in order that duplexes will form between the DPB1 DNA fragments from the sample of genomic DNA under consideration and the construct. In addition, at the known variable nucleotides of the DPB1 sequence, deliberate nucleotide substitutions (sometimes hereinbelow referred to as "identifiers" as they "identify" any mutation at this position) are created. Each of these deliberate substitutions is marked by an asterisk. The substitutions are chosen such that, in different allelic forms of the gene, a different extent of mismatch will exist. In addition, adjacent the known variable nucleotides, it may be desirable to create a deliberate mismatching with a conserved nucleotide; these are underlined in the construct sequence and effectively amplify the adjacent mismatches. As a result, hereinbelow, these are occasionally referred to as "amplifiers". The DNA construct shown has a number of different mismatches at different variable nucleotides of the consensus sequence and, by careful choice of the mismatches, different numbers and extent of single stranded loops will be formed in the duplex between the nucleotide sequence under investigation and the construct, for different alleles. In addition, a deliberate deletion, for example the 5 base pair deletion in the construct shown by the "Δ" in Figure 1 ensures that all duplexes formed between the genomic DNA derived from the sample and the construct will migrate at a significantly different rate to the duplexes formed within the population of genomic DNA. This "shifts" the bands derived from duplexes including the construct away from bands derived from duplexes not including the construct in the PCR fingerprint produced in accordance with the present invention.

The procedure to produce a PCR fingerprint for the DRB1 loci was as follows.

The DBP nucleotide sequences under investigation (second exon) were amplified in the polymerase chain reaction from genomic DNA. The PCR primers employed were those described by Begovich *et al* (Begovich AB, Bugawan TL, Nepom BS, Klitz W, Nepom GT and Erlich HA (1989), "A specific HLA-DPβ allele is associated with pauciarticular juvenile rheumatoid arthritis but not adult rheumatoid arthritis", *Proc Natl Acad Sci USA* 86:9489-9493) and which are identified as UG19 (SEQ ID NO: 6) and UG21 (SEQ ID NO: 7), available from suppliers of custom oligonucleotides. The reaction mixture contained:

| | |
|---|---|
| Genomic DNA @ 0.1µg/µl | 5.0µl |
| UG19 primer (5µM stock) | 10.0µl |
| UG21 primer (5µM stock) | 10.0µl |
| 10X PCR buffer (supplied) | 10.0µl |
| dNTP mix (10mM each DNTP) | 2.0µl |
| ddH₂O | 62.5µl |

The resulting mixtures were combined, vortexed and then centrifuged briefly. The genomic DNA was denatured by heating the tube at 94°C for 5 min and then snap cooled on ice for 3 min and overlaid with 75µl of paraffin oil.

PCR was initiated using a "hot-start" procedure, whereby tubes containing the reaction mixture without *Taq* polymerase were equilibrated at 72°C in a heat block. *Taq* polymerase (2.5 units, 0.5µl) was then added through the mineral oil, and the tube was vortexed again, recentrifuged briefly and samples immediately transferred to the thermal cycler, previously set at 72°C. The genomic DNA samples were then subjected to 35 PCR cycles, as follows.

| | |
|---|---|
| primer extension | 72°C, 30 sec |
| denaturation | 94°C, 30 sec |
| primer annealing | 65°C, 60 sec |

The 72°C primer extension time at the end of the final cycle was increased to 10 min.

The DNA construct was amplified from the M13mp18 clone using the same primers as for the genomic DNA under study, the reaction mixture being as follows:

| | |
|---|---|
| DPB-UHG M13mp18 DNA: 1/100 dilution from supplied stock | 1.0µl |
| UG19 primer (5µM stock) | 10.0µl |
| UG21 primer (5µM stock) | 10.0µl |
| 10X PCR buffer (supplied) | 10.0µl |
| dNTP mix (10mM each dNTP) | 2.0µl |
| ddH₂O | 66.5µl |

The same PCR and thermal cycling conditions were used as above for the genomic DNA, but with only 25 cycles.

Following the PCR of the construct, 5µl of PCR product was added to 1µl of 6X gel loading buffer and electrophoresed for 40 min at 70 volts in a 1.5% (w/v) agarose minigel in 1X TAE running buffer containing 0.5µg/ml ethidium bromide (Sambrook J, Fritsch EF and Maniatis T (1989) Molecular cloning, A laboratory manual; Cold Spring Harbor Laboratory Press) (Sambrook *et al* 1989, *Supra).* The resulting bands were the examined over a UV transilluminator and compared with the relative intensities of sample and DPB-construct PCRs and the concentrations adjusted to give the same intensity of fluorescence. Appropriate amounts (6-20µl) of both PCR products were added to a single microcentrifuge tube, and subjected to a further 3 PCR cycles as above. A 12µl aliquot was analysed for heteroduplexes by polyacrylamide minigel electrophoresis as described for the Mini-Protean II gel electrophoresis system (Bio-Rad). In this technique, pour a 12% nondenaturing polyacrylamide (29:1) minigel, using 1X TBE running buffer and then add 8-10µl of PCR product to 2µl 6X gel loading buffer containing bromophenol blue and xylene cyanole FF. Electrophorese at 200 volts for 90-95 min. The exact time will need to be determined experimentally: the homoduplex band should be allowed to migrate to within 0.5cm of the end of the gel. Stain gels for 20 min in 1X TBE containing 0.5µg/ml ethidium bromide and examine using a UV transilluminator.

Following this procedure PCR fingerprints for HLA-DPB1 alleles were obtained (see Figure 2).

For each HLA-DPB1 allele listed (DPB1*0101 to 2001), one of the two possible heteroduplexes formed with the DPB-construct is represented: single nucleotide mismatches are shown as boxes, matched regions as lines. For example in DPB1*0101, mismatches in complementarity between the **coding** strand of the DPB1*0101 (upper) sequence and the "**noncoding**" strand of the construct (lower) sequence are shown, and in each case the individual mismatched nucleotide on the DPB1*0101 sequence is specified (see key). Nucleotides are numbered from the 5'-end of the DPB PCR primer UG19 (SEQ ID NO: 6). Black triangles represent deliberate deletions in the DPB-construct designed to increase gel retardation of the heteroduplexes and thereby to produce a clear spread of visible bands (see Figs. 3 and 4b). The single DPB-construct employed theoretically generates an unique heteroduplex with each DPB1 allele.

In addition, PCR fingerprints of HLA-DPB1 homozygous typing cells (HTCs) conducted in the presence of the DPB-construct were obtained (see Figure 3). In each case an unique PCR fingerprint is observed. Lane 11 shows an HLA-DP heterozygote: the pattern observed is in part a combination of the relevant HTC patterns (lanes 5 and 6). M, molecular weight marker (pUCBM21 *Hpa*II plus *Dra*I and *Hind*III digests: fragment sizes 1114, 900, 692, 501, 489, 404, 320, 242, 190, 147, 124, 110, 67, 37, 34, 34, 26 and 19 base pairs).

Finally, PCR fingerprints of HLA-DPB1 heterozygotes were obtained. PCRs not conducted in the presence of the DPB-construct show (see Figure 4a) some rudimentary heteroduplexes formed in *trans* between alleles in some, but not all, combinations of alleles (lanes 3, 4, 6, and 7). The effect of conducting the PCRs as shown in (Figure 4a) in the presence of the construct is shown in Figure 4b. Unique PCR fingerprints are now revealed for all heterozygotes, with excellent resolution of heteroduplexes. Lanes 7 and 8 show a leukaemic patient and potential donor, respectively, matched for HLA-A, B and DR-Dw but mismatched for DP as revealed by the PCR fingerprints. The patient (lane 7) is DPB1*0301,0401 (compare with lane 3) and the potential donor (lane 8) is DPB1*0201,0401 (compare with lane 1). The mixed lymphocyte reaction (MLR) was positive in this case. M, molecular weight marker (see above).

### EXAMPLE 2

This example illustrates the application of the present invention to the classification of phenylketonuria genotypes.

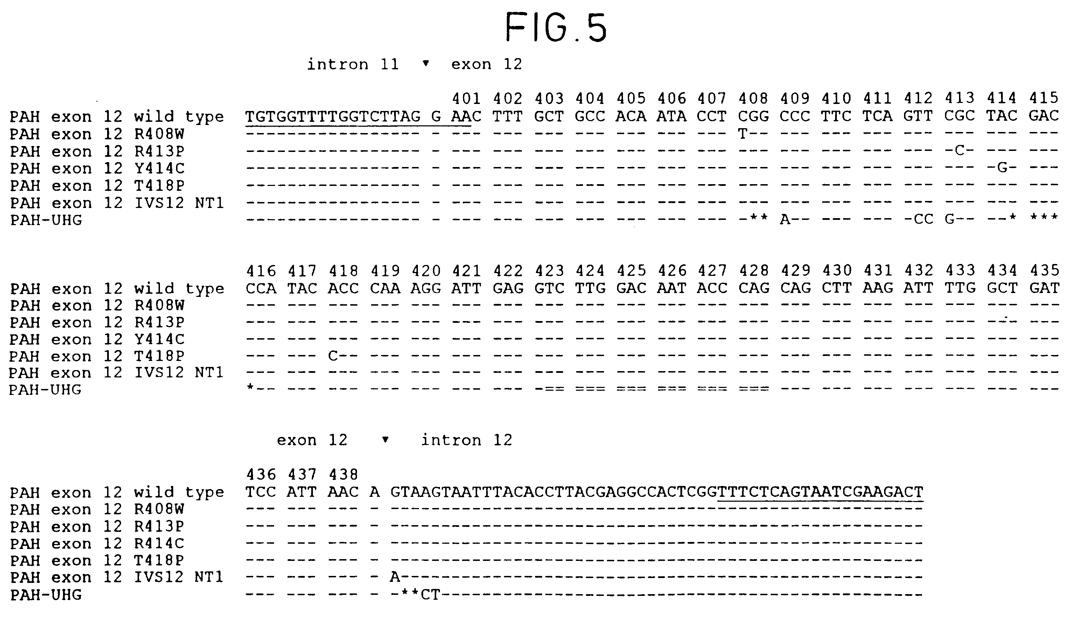

A DNA construct (SEQ ID NO: 8) was synthesised to identify five mutations (R408W, R413P, Y414C, T418P and IVS12nt1) within exon 12 of the phenylalanine hydroxylase (PAH) gene. Identifiers for four of the five mutations were incorporated within the construct and consisted of nucleotide substitutions or substitutions plus deletions. A specific identifier for the T418P mutation was not necessary. Figure 5 shows the nucleotide sequence of a portion of the normal human PAH gene, including exon 12 and part of the 5' and 3' flanking introns. Sequences of the PKU point mutations and the DNA construct are shown aligned to the normal sequence: dashes indicate identity. Annealing locations for the PAH12L and PAH12R PCR primers (see below) within the PAH gene are underlined. The overlapping complementary regions of the two constituent longmers are denoted by =. The construct (which is the lower line of Figure 5 -PAH-UHG and which is SEQ ID NO: 8 in the attached sequence listing) was synthesised from two synthetic oligonucleotides ("longmers"): PAH-LEFT #330 (sense strand), a 95mer (SEQ ID NO: 9), and PAH-RIGHT #335 (antisense strand), a 97mer (SEQ ID NO: 10), with a region of 3'-complementarity of 16 bases (Fig 5). Sequences of the longmers were: and

These were synthesized on an ABI 391A DNA synthesizer using 40 nmole polystyrene support columns (Applied Biosystems). Following synthesis, the two longmers were joined to form a double stranded construct as follows. Reaction mixtures were set up containing 2.5µl of a 5µM aqueous solution of each longmer, 5µl dNTP mix (dATP, dCTP, dGTP and dTTP each at a concentration of 10mM), 1µl of 10mg/ml bovine serum albumin, 10µl Vent^{TM} DNA polymerase reaction buffer (New England Biolabs), 58µl deionized double distilled water, 2 units Vent^{TM} DNA polymerase (New England Biolabs), added at 72°C to hot-start the reaction (D'Aquila et al, 1991), and 0.5µl E. coli single stranded DNA binding protein (Bind Aid^{TM}, United States Biochemicals). Mixtures were subjected to heating at 94°C for 1 min and cooling at 37°C for 1 min. The temperature was increased to 72°C and 10µl each of 5µM solutions of exon 12 PCR primers were added. The sequences of these primers were:

Thermal cycling conditions were as for genomic DNA amplification (see below). The products of six replicate reactions were pooled and electrophoresed on two 8% w/v nondenaturing polyacrylamide gels (Protogel, National Diagnostics) as previously described (Bidwell and Hui, 1990). Gel slices containing the double stranded construct band were excised and the DNA extracted by isotachophoresis (Ofverstedt et al, 1984), precipitated with ethanol, washed in 70% ethanol, and redissolved in 50µl sterile double distilled water. Ten microlitres of a 1:10,000 serial dilution was amplified for use in the heteroduplex analyses.

Genomic DNA was isolated from whole blood according to standard protocols, or alternatively from dried blood spots used in neonatal screening tests, as follows: a 2mm² area of blood spot was excised from the paper, added to a 0.5ml microcentrifuge tube containing 25µl of a 1:10 dilution of AmpliTaq^{TM} reaction buffer (Perkin Elmer Cetus). Samples were heated at 96°C for 15 min, cooled, diluted with a further 25µl of 1X AmpliTaq^{TM} reaction buffer, mixed and centrifuged at 13000g for 15 min. Supernatants were used directly in the PCR. Separate UHG and genomic DNA PCR amplifications were performed in 100µl reaction mixtures containing 10µl heat-denatured diluted UHG or genomic DNA isolated from whole blood (100ng) respectively, 10µl each of 5µM solutions of PAH12L and PAH12R primers (details above), 2µl dNTP mix (dATP, dCTP, dGTP and dTTP (Boehringer Mannheim) each at a concentration of 10mM), 58µl deionized double distilled water, 10µl AmpliTaq^{TM} reaction buffer and 2 units AmpliTaq^{TM} DNA polymerase (Perkin Elmer Cetus). The enzyme was added at 72°C to hot-start the reaction (D'Aquila et al, 1991). For PCR amplifications of DNA obtained from dried blood spots, 30µl of supernatant was used in the reaction mixtures, and the volumes of AmpliTaq^{TM} reaction buffer and water were reduced to 7µl and 40.6p1 respectively. The reaction mixtures were subjected to 35 rounds of thermal cycling at 94°C for 1 min, 48°C for 1 min and 72°C for 1 min.

Fifteen microlitres of PCR-amplified genomic DNA and of PCR-amplified construct were mixed and subjected to 3 rounds of thermal cycling at 94°C for 1 min, 65°C for 2.5 min and 72°C for 2.5 min (Note: 1 amplification of the construct was therefore sufficient for 6 reactions with PCR-amplified genomic DNA). The entire mixture was subjected to electrophoresis for 90 min at 200V on 12% w/v nondenaturing polyacrylamide minigels (Protogel, National Diagnostics) and heteroduplexes were visualised by staining with ethidium bromide as previously described (Bidwell and Hui, 1990).

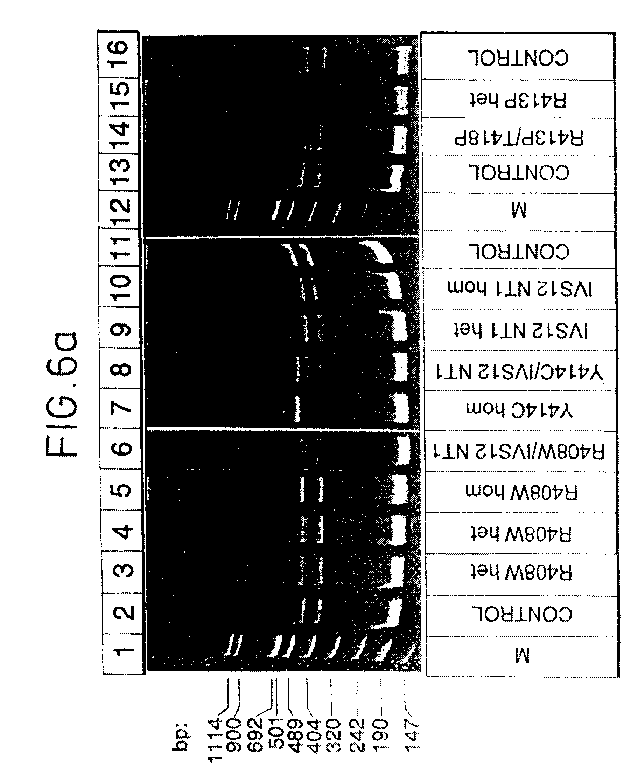

Figure 6a, 6b, 7 and 8 show the stained gels for a series of experiments on the of PAH gene exon 12 mutations.

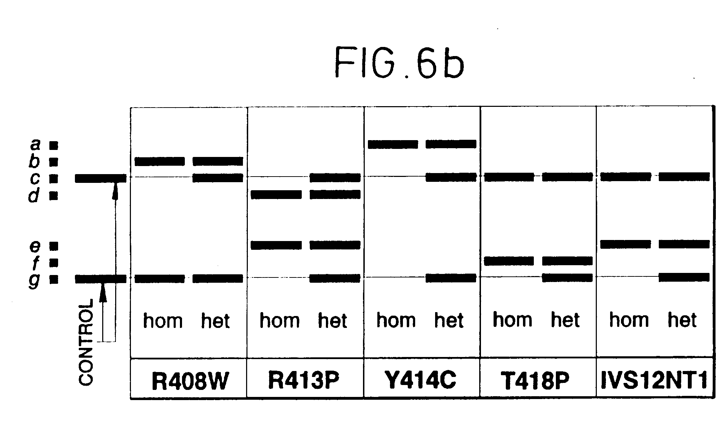

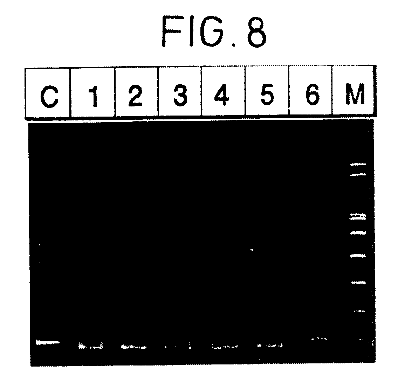

In Figure 6(a), Lanes 1 and 12 show molecular weight markers (M, marker VIII, Boehringer Mannheim). The leading double bands in all other lanes are the homoduplexes of the synthetic DNA construct (leading band, 176bp) and PAH gene exon 12 (trailing band, 185bp) PCR products. Bands of apparent molecular weight 400-500bp are different conformational forms of DNA heteroduplexes (Figs 6b and 8). Lanes 2, 11, 13 and 16 show the heteroduplexes observed in normal individuals (controls). Other lanes show the variations in heteroduplexes observed in PAH gene exon 12 mutations, denoted beneath each lane: hom, individual homozygous for mutation; het, individual heterozygous for an exon 12 mutation (non-exon 12 PAH gene mutation present on other chromosome). Lanes 6, 8 and 14 show individuals with different PAH gene exon 12 mutations on each chromosome. In all cases, characteristic heteroduplexes (also see Figure 6b) are observed for each mutation, and in addition the homozygosity or heterozygosity status is readily determined.

Figure 6(b) is a schematic representation of DNA heteroduplex banding patterns obtained for each PAH gene exon 12 mutation. For each mutation, banding patterns are shown for homozygous (hom) and heterozygous (het) states. The mobilities of the bands (apparent molecular weights) are: a, 446bp; b, 433bp; c, 416bp; d, 395bp; e, 374bp; f, 359bp; g, 354bp. Bands c plus g are observed in normal PAH genes and in genes with non-exon 12 mutations.

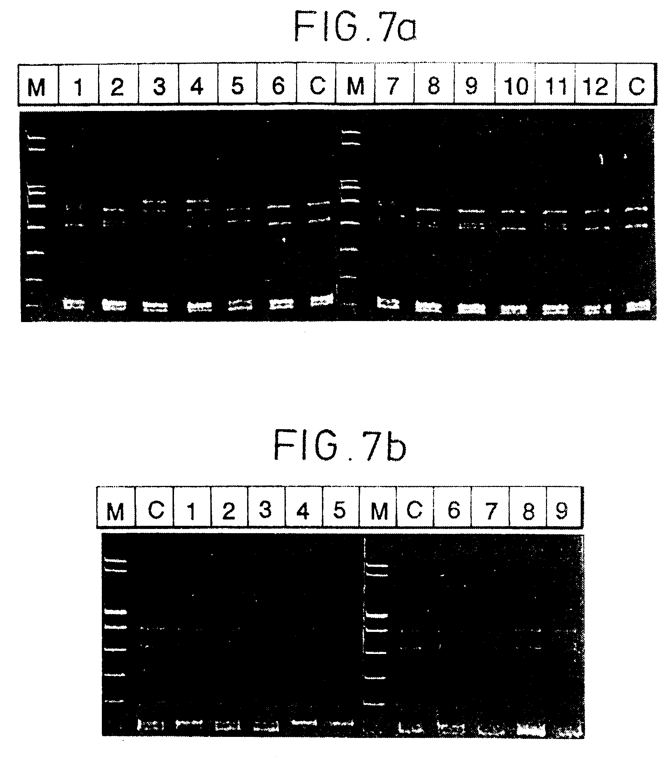

The results shown in Figure 8 were obtained in the same way as Figures 7a and 7b and show the classification of mutations in PKU individuals using DNA isolated from dried blood spots. C, normal control; M, molecular weight markers (for details see Fig 2 legend); lane 1, individual with R408W and IVS12nt1 mutations on opposite alleles; lanes 2, 3, 5 and 6, IVS12nt1 heterozygous individuals; lane 4, IVS12nt1 homozygous individual.

Figures 6a and 6b and 8 show that the synthetic construct was able to generate unique and discriminatory DNA heteroduplexes for all five known exon 12 mutations within DNA from PKU individuals, both in the homozygous and heterozygous states. Moreover, the visual quality of these results compared favourably with those obtained with DNA isolated from fresh blood samples, and permitted unambiguous assignment of exon 12 genotypes.

The reliability and reproducibility of the test was examined by screening two sets of genomic DNA samples collected from PKU individuals in whom exon 12 mutations were established by other methods, either prospectively or retrospectively.

Figures 7a and 7b show the results of a blind trial of concordance between the DNA analysis method of the present invention and the results of other DNA typing analyses. Figure 7a represents the results using the first set of genomic DNA whilst Figure 7b represents the results using the second set of genomic DNA constituting untreated, PKU individuals with reduced intelligence from a Scottish population. In both Figures 7a and 7b, lane M are molecular weight markers (for details see Figure 6 legend) and lane C is a normal control. In Figure 7a, the lanes were as follows: lane 1, R408W heterozygous individual; lanes 2, 8, 9 and 11, IVS12nt1 heterozygous individuals; plane 3, individual with Y414C and IVS12nt1 mutations on opposite alleles; lane 4, Y414C heterozygous individual; lane 5, IVS12nt1 homozygote; lane 7, individual with R408W and IVS12nt1 mutations on opposite alleles. Lanes 6, 10 and 12 are PKU individuals with mutations outside exon 12. In Figure 7b, the lanes are as follows: lanes 1-7 and 9, IVS12nt1 heterozygous individuals; lane 8, PKU individual with mutation outside exon 12. For the first set of genomic DNA (Figure 7a) there was complete concordance between results of the PAH-UHG tests and prospectively performed analyses using allele specific oligonucleotide (ASO) probes or DNA sequencing.

In the second set of genomic DNA, constituting untreated, PKU individuals with reduced intelligence from a Scottish population, 8 out of 9 samples were identified as heterozygous for the IVS12nt1 mutation (Figure 3b); these results were confirmed by ASO analysis (data not shown), and are not unexpected for this population (Sullivan et al, 1989).

A practical consideration of the potential application of this technology is in the ability to rapidly genotype infants who are positively diagnosed in the neonatal screen for PKU. We addressed this by analysing genomic DNA prepared from a 2mm² area of dried blood spots normally used in the neonatal screening programme. These samples had been obtained from affected individuals who regularly send dried blood spots to the screening laboratory for monitoring blood phenylalanine concentrations. The samples were between 1 to 4 months old and had been left at ambient temperatures during that time. Samples were analysed from PKU individuals whose genotype had been established previously using ASO probes or DNA sequencing. In all cases, the visual quality of these results (Figure 8) compared favourably with those obtained with DNA isolated from fresh blood samples, and permitted unambiguous assignment of exon 12 genotypes.

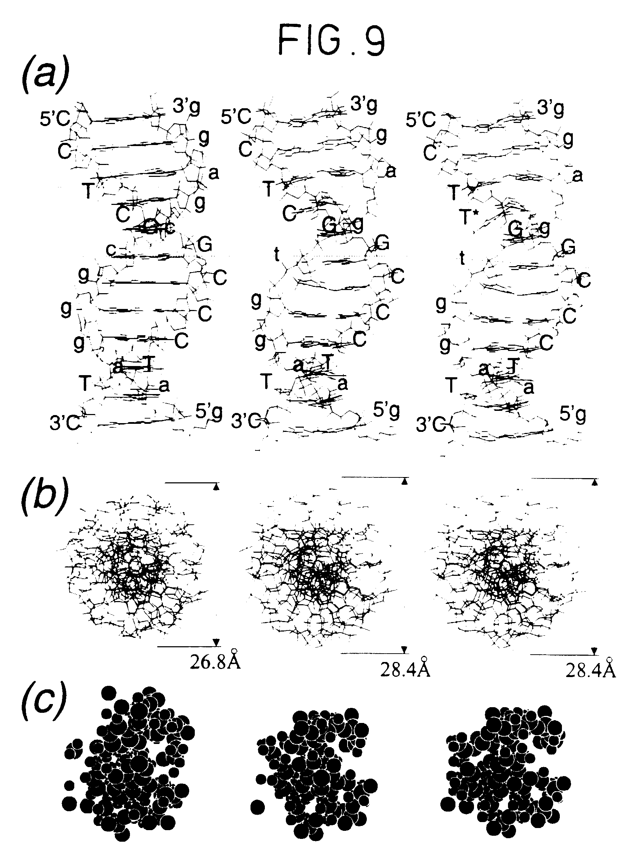

This Example shows the technique of the present invention to be a highly sensitive, rapid and non-radioactive screening and classification system for PKU in which five mutations in exon 12 of the PAH gene can be identified using a single DNA construct and one PCR-based test. The tests are technically simple to perform and suitable for the routine screening of large numbers of individuals. Results were obtained within 24 hours of receipt of blood samples, and were of equal visual quality when performed on whole blood (Figures 6 and 7) or dried blood spots (Figure 8). Evidence from this and parallel studies being conducted for other genetic diseases, suggests that previously undescribed mutations within sequences under study may be identified initially by an unmodified DNA construct. Following DNA sequence analysis to identify the new mutation, specific modifications to the construct, derived from molecular modelling simulations, can optimise the identification of the new mutation in subsequent tests. The conformational and surface charge modifications induced in heteroduplexed DNA by constructs may be modelled. An example of the homoduplexed and synthetic construct-heteroduplexed nucleotides between codons 407 and 410 of exon 12 of the PAH gene using in the model the construct sequence which identifies the R408W mutation is shown in Figure 9. Conformational differences include altered interatomic distances and angles, increased diameter of the helix (compare diameters in (b)), and changes in net surface charge. The changes in electrophoretic mobilities as a consequence of such modifications may be predicted to a large extent from such theoretical models prior to actual construction of specific synthetic constructs. This Example illustrates the wide potential of synthetic DNA construct-based DNA heteroduplex technology. The technique appears ideally suited to the genotyping of multi-allelic genes where mutations are clustered within a reasonable distance and are flanked by conserved sequences suitable for PCR priming. Genotyping mutations within separate exons in genomic DNA may be facilitated using exon-specific constructs, or potentially by a single construct for genotyping cDNA derived from mRNA by reverse transcription PCR (Myers and Gelfand, 1991)

### BIBLIOGRAPHY

D'Aquila RT, Bechtel RJ, Videler JA, Eron JJ, Gorczyca P, Kaplan JC (1991) Maximizing sensitivity and specificity of PCR by pre-amplification heating. Nucl Acids Res , 19:3749.
Begovich AB, Bugawan TL, Nepom BS, Klitz W, Nepom GT and Erlich HA (1989) A specific HLA-DPβ allele is associated with pauciarticular juvenile rheumatoid arthritis but not adult rheumatoid arthritis. Proc Natl Acad Sci USA 86:9489-9493
Bidwell JL and Hui KM (1990) Human HLA-DR-Dw allotype matching by analysis of HLA-DRB gene PCR product polymorphism (PCR "fingerprints"). Technique 2:93-100
Bidwell JL, Clay TM, Wood NAP, Pursall MP, Martin AF, Bradley BA, Hui KM (1992) Rapid HLA-DR-Dw and DP matching by PCR fingerprinting and related DNA heteroduplex technologies. In Hui KM, Bidwell JL (eds): Handbook of HLA Typing Techniques. New York: CRC Press, Inc., (in press).
Bidwell JL (1992) Selecting UD-BMT donors by PCR fingerprinting EBMT News 1:6-7
Cotton RGH, Rodrigues NR, Campbell RD (1988) Reactivity of cytosine and thymine in single-base-pair mismatches with hydroxylamine and osmium tetroxide and its application to the study of mutations. Proc Natl Acad Sci USA 85:4397-4401.
Clay TM, Bidwell JL, Howard MR and Bradley BA (1991) PCR-fingerprinting for selection of HLA-matched unrelated marrow donors. Lancet 337:1049-1052
DiLella AG, Kwok SCM, Ledley FD, Marvit J, Woo SLC (1986) Molecular structure and polymorphic map of the human phenylalanine hydroxylase gene. Biochemistry 25:743-749.
Dworniczak B, Aulehla-Scholz C, Kalaydjieva L, Bartholome K, Grudda K, Horst J (1991) Aberrant splicing of phenylalanine hydroxylase mRNA: The major cause of phenylketonuria in parts of Southern Europe. Genomics 11:242-246.
Fisher SG, Lerman LS (1983) DNA fragments differing by single-base pair substitutions are separated in denaturing gradient gel: correspondence with melting theory. Proc Natl Acad Sci USA 80: 1579-1583.
John SWM, Scriver CR, Laframboise R, Rozen R (1992) In vitro and in vivo correlations for I65T and M1V mutations at the phenylalanine hydroxylase locus. Hum Mut 1:147-153.
Kwok SCM, Ledley FD, DiLella AG, Robson KJH, Woo SLC (1985) Nucleotide sequence of a full-length complemeentary DNA clone and amino acid sequence of human phenylalanine hydroxylase. Biochemistry 24:556-561.
Lou HC, Toft PB, Andresen J, Mikkelsen I, Olsen B, Guttler F, Wieslander S, Henriksen O (1992) An occipito-temporal syndrome in adolescents with optimally controlled hyperphenylalaninaemia. J Inher Metab Dis 15:687-695.
Myers TW, Gelfand DH (1991) Reverse transcription and DNA amplification by a Thermus thermophilus DNA polymerase. Biochemistry 30:7661-7666.
Ofverstedt L-G, Hammarstrom K, Balgobin M, Hjerten S, Pettersson U, Chattopadhyaya J (1984) Rapid and quantitative recovery of DNA fragments from gels by displacement electrophoresis (isotachophoresis). Biochim Biophys Acta 782:120-126.
Okano Y, Eisensmith RC, Dasovich M, Wang T, Guttler F, Woo SLC (1991a) A prevalent missense mutation in Northern Europe associated with hyperphenylalaninaemia. Eur J Pediatr 150:347-352.
Okano Y, Eisensmith RC, Guttler F, Lichter-Konecki U, Konecki D, Trefz FK, Dasocich M, Wang T, Henriksen K, Lou H, Woo SLC (1991b) Molecular basis of phenotypic heterogeneity in phenylketonuria. N Eng J Med 324:1232-1238.
Orita M, Suzuki Y, Sekiya T, Hayashi K (1989) Rapid and sensitive detection of point mutations and DNA polymorphisms using the polymerase chain reaction. Genomics 5:874-879.
Sambrook J, Fritsch EF and Maniatis T (1989) Molecular cloning. A laboratory manual. Cold Spring Harbor Laboratory Press.
Scharf S, Long C and Erlich H (1988) Sequence analysis of HLA-DR and HLA-DQ loci from three Pemphigus vulgaris patients. Human Immunol 22:61-69
Scriver CR, John SMW, Rozen R, Eisensmith RC, Woo SLC (1992) Associations between population, PKU mutations and RFLP haplotypes at the PAH locus. Brain Dysfunction (in the press).
Sullivan SE, Moore SD, Connor JM, King M, Cockburn F, Steinmann B, Gitzelmann R, Daiger SP, Woo SLC (1989) Haplotype distribution of the human phenylalanine hydroxylase locus in Scotland and Switzerland. Am J Hum Genet 44:652-659.
Thompson AJ, Smith I, Kendall BE, Youl BD, Brenton D (1991) MRI changes in early treated patients with phenylketonuria. Lancet 337:1224.
Tyfield LA, Osborn MJ, Holton JB (1991) Molecular heterogeneity at the phenylalanine hydroxylase locus in the population of the South-west of England. J Med Genet 28:244-247.
Wood NAP, Clay TM and Bidwell JL (1991) HLA-DR-Dw matching by PCR fingerprinting: the origin of PCR fingerprints and further applications. Eur J Immunogenet 18:147-153

## Claims

1. A method for examining polymorphisms in a nucleotide sequence of a first sample of nucleic acid comprising the steps of:
(a) forming a population of nucleic acid fragments bearing the said nucleotide sequence;
(b) combining the population of nucleic acid fragments with a population of a synthetic nucleotide construct which construct is capable of forming duplexes with the nucleic acid fragments, the sequence of the construct being such that duplexes of different molecular conformation are formed between the construct and the nucleic acid fragments dependent upon the presence of a polymorphism at a known variable nucleotide or sequence of nucleotides within the sequence of the sample under examination;
(c) permitting duplex formation within the combined populations; and
(d) separating the duplexes formed in step (c);
wherein the synthetic construct contains one or more deliberate nucleotide substitution(s) and/or deletion(s) and/or insertion(s) which is/are (i) opposite to a known variable nucleotide or sequence of nucleotides within the sequence under examination and/or (ii) contiguous with a nucleotide which is opposite to a known variable nucleotide or sequence of nucleotides within the sequence under examination.

2. A method according to claim 1, wherein the nucleotide sequence of the synthetic construct at least contains deliberate nucleotide substitution(s) and/or deletion(s) and/or insertion(s) contiguous with the nucleotide which is opposite the known variable nucleotide or sequence of nucleotides within the sequence under examination.

3. A method according to claim 1 or 2, wherein the nucleotide sequence of the synthetic construct contains deliberate nucleotide substitution(s) and/or deletion(s) and/or insertion(s) opposite the known variable nucleotide or sequence of nucleotides.

4. A method according to claim 3, wherein the synthetic construct includes nucleotide substitution(s) and deletion(s) both opposite and contiguous with the mutation.

5. A method according to any one of claims 1 to 4, wherein the nucleotide sequence of the synthetic construct contains between one and ten contiguous substitutions/deletions adjacent the position of the polymorphism in the nucleotide sequence under investigation

6. A method according to any one of claims 1 to 5, wherein the synthetic construct includes deliberate nucleotide substitution(s) and/or deletion(s) and/or insertion(s) corresponding to at least two known polymorphisms in the nucleotide sequence

7. A method according to any preceding claim, wherein the construct includes a deliberate deletion or a series of deletions, at a position away from polymorphic sites in the nucleotide sequence under investigation.

8. A method according to any preceding claim, wherein the nucleic acid under examination is DNA.

9. A method according to any preceding claim, wherein the synthetic nucleotide construct is a synthetic DNA.

10. A method according to any preceding claim, wherein the population of DNA fragments bearing the nucleotide sequence is formed from a DNA source by a polymerase chain reaction.

11. A method according to any preceding claim in which the synthetic construct is a DNA construct, wherein the population of synthetic DNA construct is formed by a polymerase chain reaction.

12. A method according to any preceding claim for matching individuals in respect of one or more of the functional proteins expressed by the HLA (human leucocyte antigen) class I and II genes, wherein the nucleotide sequence under examination is a sequence, or part of a sequence, of a HLA Class I or II gene containing a known polymorphic site or sites.

13. A method according to any one of claims 1 to 11, for determining an individual's susceptibility to a genetic disease, wherein the nucleotide sequence under examination is a sequence, or part of a sequence, of a gene containing a known polymorphic site or sites associated with a genetic defect.

14. A method according to claim 13, wherein the genetic disease is phenylketonuria genotyping and the gene containing a known polymorphic site or sites is the phenylalanine hydroxylase (PAH) gene.

15. A test kit, which kit comprises:
(a) two oligonucleotide primers suitable for use in PCR and capable of annealing to complementary sequences at respective ends of a nucleotide sequence to be examined;
(b) a synthetic nucleotide construct which construct is capable of forming duplexes with the nucleotide sequence under consideration, the sequence of the construct being such that duplexes of different molecular conformation are formed between the construct and the nucleotide sequence under examination dependent upon the presence of a polymorphism at a known variable nucleotide or sequence of nucleotides within the sequence under examination; and
(c) a control DNA and/or control PCR amplification product;
wherein the synthetic construct contains one or more deliberate nucleotide substitution(s) and/or deletion(s) and/or insertion(s) which is/are (i) opposite to a known variable nucleotide or sequence of nucleotides within the sequence under examination and/or (ii) contiguous with a nucleotide which is opposite to a known variable nucleotide or sequence of nucleotides within the sequence under examination.

16. A test kit according to claim 15, wherein the synthetic nucleotide construct is a synthetic DNA.

## Patentansprüche

1. Verfahren zur Untersuchung von Polymorphismen in einer Nucleotidsequenz einer ersten Nucleinsäureprobe, umfassend die Schritte:
(a) Bilden einer Population aus Nucleinsäurefragmenten, die die Nucleotidsequenz enthalten,
(b) Vereinen der Population aus Nucleinsäurefragmenten mit einer Population eines synthetischen Nucleotidkonstrukts, das mit den Nucleinsäurefragmenten Duplexe bilden kann, wobei die Sequenz des Konstrukts so ist, dass Duplexe mit verschiedenen Molekülkonformationen gebildet werden können zwischen dem Konstrukt und den Nucleinsäurefragmenten, abhängig vom Vorliegen eines Polymorphismus an einem bekannten variablen Nucleotid oder einer bekannten variablen Nucleotidsequenz in der Sequenz der untersuchten Probe,
(c) Zulassen der Duplexbildung innerhalb der vereinten Populationen, und
(d) Trennen der in Schritt (c) gebildeten Duplexe, wobei das synthetische Konstrukt ein oder mehrere gezielte Nucleotid-Substitutionen, -Deletionen und/oder -Insertionen enthält, die (i) gegenüber einem bekannten variablen Nucleotid oder einer bekannten variablen Nucleotidsequenz sind in der untersuchten Sequenz und/oder (ii) nebst einem Nucleotid sind, das einem bekannten variablen Nucleotid oder einer bekannten variablen Nucleotidsequenz gegenüber ist in der untersuchten Sequenz.

2. Verfahren nach Anspruch 1, wobei die Nucleotidsequenz des synthetischen Konstrukts mindestens ein oder mehrere gezielte Nucleotid-Substitutionen, -Deletionen und/oder -Insertionen enthält nebst dem Nucleotid, das gegenüber dem bekannten variablen Nucleotid oder der bekannten variablen Nucleotidsequenz ist in der untersuchten Sequenz.

3. Verfahren nach Anspruch 1 oder 2, wobei die Nucleotidsequenz des synthetischen Konstrukts ein oder mehrere gezielte Nucleotid-Substitutionen, -Deletionen und/oder -Insertionen enthält, gegenüber dem bekannten variablen Nucleotid oder der bekannten variablen Nucleotidsequenz.

4. Verfahren nach Anspruch 3, wobei das synthetische Konstrukt ein oder mehrere Nucleotid-Substitutionen und -Deletionen enthält, sowohl gegenüber als auch nebst der Mutation.

5. Verfahren nach einem der Ansprüche 1 bis 4, wobei die Nucleotidsequenz des synthetischen Konstrukts zwischen 1 und 10 angrenzende Substitutionen/Deletionen enthält nebst der Position des Polymorphismus in der untersuchten Nucleotidsequenz.

6. Verfahren nach einem der Ansprüche 1 bis 5, wobei das synthetische Konstrukt ein oder mehrere gezielte Nucleotid-Substitutionen, -Deletionen und/oder -Insertionen enthält, die mindestens zwei bekannten Polymorphismen in der Nucleotidsequenz entsprechen.

7. Verfahren nach einem der vorstehenden Ansprüche, wobei das Konstrukt eine gezielte Deletion enthält oder eine Reihe von Deletionen an einer Stelle, entfernt von den polymorphen Bereichen in der untersuchten Nucleotidsequenz.

8. Verfahren nach einem der vorstehenden Ansprüche, wobei die untersuchte Nucleinsäure DNS ist.

9. Verfahren nach einem der vorstehenden Ansprüche, wobei das synthetische Nucleotidkonstrukt eine synthetische DNS ist.

10. Verfahren nach einem der vorstehenden Ansprüche, wobei die Population aus DNS-Fragmenten, die die Nucleotidsequenz enthalten, durch eine Polymerasekettenreaktion aus einer DNS-Quelle gebildet wird.

11. Verfahren nach einem der vorstehenden Ansprüche, bei dem das synthetische Konstrukt ein DNS-Konstrukt ist, wobei die Population aus synthetischem DNS-Konstrukt durch eine Polymerasekettenreaktion gebildet wird.

12. Verfahren nach einem der vorstehenden Ansprüche zum Zuordnen von Individuen bezüglich ein oder mehrerer funktionaler Proteine, die von HLA (Human-Leucocyten-Antigen)-Genen der Klasse I und II exprimiert werden, wobei die untersuchte Nucleotidsequenz eine Sequenz oder ein Sequenzteil eines HLA-Gens der Klasse I oder II ist, das den oder die bekannten polymorphen Bereiche enthält.

13. Verfahren nach einem der Ansprüche 1 bis 11, zur Feststellung, ob eine Person eine Veranlagung für eine genetische Krankheit hat, wobei die untersuchte Nucleotidsequenz eine Sequenz oder ein Sequenzteil eines Gens ist, das ein oder mehrere bekannte polymorphe Bereiche enthält, die mit einem Gendefekt verbunden sind.

14. Verfahren nach Anspruch 13, wobei die genetische Krankheit der Phenylketonurie-Genotyp ist und das Gen, das ein oder mehrere bekannte polymorphe Bereiche enthält, das Phenylalanin-Hydroxylase(PAH)-Gen ist.

15. Ein Testkit, umfassend
(a) zwei Oligonucleotid-Primer, geeignet zur Verwendung in der PCR und zum Tempern an komplementäre Sequenzen an den jeweiligen Enden einer untersuchten Nucleotidsequenz,
(b) ein synthetisches Nucleotidkonstrukt, das Duplexe mit der untersuchten Nucleotidsequenz bilden kann, wobei die Sequenz des Konstrukts so ist, dass Duplexe verschiedener Molekülkonformationen gebildet werden können zwischen dem Konstrukt und der untersuchten Nucleotidsequenz, abhängig vom Vorliegen eines Polymorphismus an einem bekannten variablen Nucleotid oder einer bekannten variablen Nucleotidsequenz in der untersuchten Sequenz, und
(c) ein Kontroll-DNS- und/oder Kontroll-PCR-Amplifikationsprodukt, wobei das synthetische Konstrukt ein oder mehrere gezielte Nucleotid-Substitutionen, -Deletionen und/oder -Insertionen enthält, die (i) einem bekannten variablen Nucleotid oder einer bekannten variablen Nucleotidsequenz gegenüber sind in der untersuchten Sequenz und/oder (ii) nebst einem Nucleotid sind, das einem bekannten variablen Nucleotid oder einer bekannten variablen Nucleotidsequenz gegenüber ist in der untersuchten Sequenz.

16. Ein Test-Kit nach Anspruch 15, wobei das synthetische Nucleotidkonstrukt eine synthetische DNS ist.

## Revendications

1. Procédé d'examen des polymorphismes dans une séquence nucléotidique d'un premier échantillon d'acide nucléique, comprenant les étapes consistant à :
(a) former une population de fragments d'acide nucléique portant ladite séquence nucléotidique ;
(b) combiner la population de fragments d'acide nucléique avec une population d'un produit d'assemblage nucléotidique de synthèse, lequel produit d'assemblage est capable de former des duplex avec les fragments d'acide nucléique, la séquence du produit d'assemblage étant telle que des duplex de conformation moléculaire différente sont formés entre le produit d'assemblage et les fragments d'acide nucléique en fonction de la présence d'un polymorphisme sur un nucléotide ou une séquence de nucléotides variables connus au sein de la séquence de l'échantillon en cours d'examen,
(c) permettre la formation de duplex au sein des populations combinées ; et
séparer les duplex formés à l'étape (c) ;
étant entendu que le produit d'assemblage de synthèse contient une ou plusieurs substitutions et/ou délétions et/ou insertions nucléotidiques délibérées qui sont (i) opposées à un nucléotide ou une séquence de nucléotides variables connus au sein de la séquence en cours d'examen et/ou (ii) contiguës à un nucléotide qui est opposé à un nucléotide ou une séquence de nucléotides variables connus au sein de la séquence en cours d'examen.

2. Procédé selon la revendication 1, où la séquence nucléotidique du produit d'assemblage de synthèse contient au moins une ou des substitutions et/ou délétions et/ou insertions nucléotidiques délibérées contiguës au nucléotide qui est opposé au nucléotide ou à la séquence de nucléotides variables connus au sein de la séquence en cours d'examen.

3. Procédé selon la revendication 1 ou 2, où la séquence nucléotidique du produit d'assemblage de synthèse contient une ou des substitutions et/ou délétions et/ou insertions nucléotidiques délibérées opposées au nucléotide ou à la séquence de nucléotides variables connus.

4. Procédé selon la revendication 3, où le produit d'assemblage de synthèse comprend une ou des substitutions et une ou des délétions nucléotidiques opposées et contiguës à la mutation.

5. Procédé selon l'une quelconque des revendications 1 à 4, où la séquence nucléotidique du produit d'assemblage de synthèse contient entre 1 et 10 substitutions/délétions contiguës, adjacentes à la position du polymorphisme dans la séquence nucléotidique en cours d'étude.

6. Procédé selon l'une quelconque des revendications 1 à 5, où le produit d'assemblage de synthèse comprend une ou des substitutions et/ou délétions et/ou insertions nucléotidiques délibérées correspondant à au moins deux polymorphismes connus dans la séquence nucléotidique.

7. Procédé selon l'une quelconque des revendications précédentes, où le produit d'assemblage contient une délétion ou une série de délétions délibérées à une position distante de sites polymorphes dans la séquence nucléotidique en cours d'étude.

8. Procédé selon l'une quelconque des revendications précédentes, où l'acide nucléique en cours d'examen est l'ADN.

9. Procédé selon l'une quelconque des revendications précédentes, où le produit d'assemblage nucléotidique de synthèse est un ADN de synthèse.

10. Procédé selon l'une quelconque des revendications précédentes, où la population de fragments d'ADN portant la séquence nucléotidique est formée à partir d'une source d'ADN par amplification en chaîne par polymérase.

11. Procédé selon l'une quelconque des revendications précédentes, où le produit d'assemblage de synthèse est un produit d'assemblage d'ADN où la population de produit d'assemblage d'ADN de synthèse est formée par amplification en chaîne par polymérase.

12. Procédé selon l'une quelconque des revendications précédentes, destiné à apparier des individus en fonction d'une ou plusieurs des protéines fonctionnelles exprimées par les gènes de HLA (antigène leucocytaire humain) de classe I et II, où la séquence nucléotidique en cours d'examen est une séquence ou une partie d'une séquence d'un gène de HLA de classe I ou II contenant un ou des sites polymorphes connus.

13. Procédé selon l'une quelconque des revendications 1 à 11, destiné à déterminer la sensibilité d'un individu à une maladie génétique, où la séquence nucléotidique en cours d'examen est une séquence ou une partie d'une séquence d'un gène contenant un ou des sites polymorphes connus associés à une anomalie génétique.

14. Procédé selon la revendication 13, où la maladie génétique est le génotypage de la phénylcétonurie et le gène contenant un ou des sites polymorphes connus est le gène de la phénylalanine hydroxylase (PAH).

15. Kit de dosage, lequel kit comprend :
(a) deux amorces oligonucléotidiques utilisables en PCR et capables de s'hybrider à des séquences complémentaires aux extrémités respectives d'une séquence nucléotidique à examiner;
(b) un produit d'assemblage nucléotidique de synthèse, lequel produit d'assemblage est capable de former des duplex avec la séquence nucléotidique considérée, la séquence du produit d'assemblage étant telle que des duplex de conformation moléculaire différente sont formés entre le produit d'assemblage et la séquence nucléotidique en cours d'examen en fonction de la présence d'un polymorphisme sur un nucléotide ou une séquence de nucléotides variables connus au sein de la séquence en cours d'examen ; et
(c) un ADN témoin et/ou un produit d'amplification par PCR témoin ;
étant entendu que le produit d'assemblage de synthèse contient une ou plusieurs substitutions et/ou délétions et/ou insertions nucléotidiques délibérées qui sont (i) opposées à un nucléotide ou une séquence de nucléotides variables connus au sein de la séquence en cours d'examen et/ou (ii) contiguës à un nucléotide qui est opposé à un nucléotide ou une séquence de nucléotides variables connus au sein de la séquence en cours d'examen.

16. Kit de dosage selon la revendication 15, où le produit d'assemblage nucléotidique de synthèse est un ADN de synthèse.


### SEQUENCE LISTING

### (1) GENERAL INFORMATION:

(i) APPLICANT:
   (A) NAME: The University of Bristol
   (B) STREET: Senate House, Tyndall Road
   (C) CITY: Bristol
   (E) COUNTRY: United Kingdom
   (F) POSTAL CODE (ZIP): BS8 1TH
(ii) TITLE OF INVENTION: NUCLEIC ACID ANALYSIS
(iii) NUMBER OF SEQUENCES: 12
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)

### (2) INFORMATION FOR SEQ ID NO: 1:

(i) SEQUENCE CHARACTERISTICS:
   (A) LENGTH: 333 base pairs
   (B) TYPE: nucleic acid
   (C) STRANDEDNESS: double
   (D) TOPOLOGY: linear
(xi) SEQUENCE DESCRIPTION: SEQ ID NO: 1:

### (2) INFORMATION FOR SEQ ID NO: 2:

(i) SEQUENCE CHARACTERISTICS:
   (A) LENGTH: 90 base pairs
   (B) TYPE: nucleic acid
   (C) STRANDEDNESS: single
   (D) TOPOLOGY: linear
(xi) SEQUENCE DESCRIPTION: SEQ ID NO: 2:

### (2) INFORMATION FOR SEQ ID NO: 3:

(i) SEQUENCE CHARACTERISTICS:
   (A) LENGTH: 92 base pairs
   (B) TYPE: nucleic acid
   (C) STRANDEDNESS: single
   (D) TOPOLOGY: linear
(xi) SEQUENCE DESCRIPTION: SEQ ID NO: 3:

### (2) INFORMATION FOR SEQ ID NO: 4:

(i) SEQUENCE CHARACTERISTICS:
   (A) LENGTH: 91 base pairs
   (B) TYPE: nucleic acid
   (C) STRANDEDNESS: single
   (D) TOPOLOGY: linear
(xi) SEQUENCE DESCRIPTION: SEQ ID NO: 4:
   GGAGTTCCGG GCGGTGACGG AGCTGGGGCG GCCGCATGAG GACAACTGGA ACAGCCAGAA 60
   GGCACTCCTG GAAGCGGGCA GTGCCGGACA G 91

### (2) INFORMATION FOR SEQ ID NO: 5:

(i) SEQUENCE CHARACTERISTICS:
   (A) LENGTH: 90 base pairs
   (B) TYPE: nucleic acid
   (C) STRANDEDNESS: single
   (D) TOPOLOGY: linear
(xi) SEQUENCE DESCRIPTION: SEQ ID NO: 5:
   CGGATCCGGC CCAAAGCCCT CACTCACCTC GGCGCTGCAG GGTCACGGCA TCGTCCAGCT 60
   CGTAGTTGTG TCTGCATTCC CTGTCCGGCA 90

### (2) INFORMATION FOR SEQ ID NO: 6:

(i) SEQUENCE CHARACTERISTICS:
   (A) LENGTH: 28 base pairs
   (B) TYPE: nucleic acid
   (C) STRANDEDNESS: single
   (D) TOPOLOGY: linear
(xi) SEQUENCE DESCRIPTION: SEQ ID NO: 6:
   GCTGCAGGAG AGTGGCGCCT CCGCTCAT 28

### (2) INFORMATION FOR SEQ ID NO: 7:

(i) SEQUENCE CHARACTERISTICS:
   (A) LENGTH: 25 base pairs
   (B) TYPE: nucleic acid
   (C) STRANDEDNESS: single
   (D) TOPOLOGY: linear
(xi) SEQUENCE DESCRIPTION: SEQ ID NO: 7:
   CGGATCCGGC CCAAAGCCCT CACTC
   25

### (2) INFORMATION FOR SEQ ID NO: 8:

(i) SEQUENCE CHARACTERISTICS:
   (A) LENGTH: 180 base pairs
   (B) TYPE: nucleic acid
   (C) STRANDEDNESS: double
   (D) TOPOLOGY: linear
(xi) SEQUENCE DESCRIPTION: SEQ ID NO: 8:

### (2) INFORMATION FOR SEQ ID NO: 9:

(i) SEQUENCE CHARACTERISTICS:
   (A) LENGTH: 95 base pairs
   (B) TYPE: nucleic acid
   (C) STRANDEDNESS: single
   (D) TOPOLOGY: linear
(xi) SEQUENCE DESCRIPTION: SEQ ID NO: 9:

### (2) INFORMATION FOR SEQ ID NO: 10:

(i) SEQUENCE CHARACTERISTICS:
   (A) LENGTH: 97 base pairs
   (B) TYPE: nucleic acid
   (C) STRANDEDNESS: single
   (D) TOPOLOGY: linear
(xi) SEQUENCE DESCRIPTION: SEQ ID NO: 10:

### (2) INFORMATION FOR SEQ ID NO: 11:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(xi) SEQUENCE DESCRIPTION: SEQ ID NO: 11:
   TGTGGTTTTG GTCTTAGGAA
                      20

### (2) INFORMATION FOR SEQ ID NO: 12:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(xi) SEQUENCE DESCRIPTION: SEQ ID NO: 12:
   AGTCTTCGAT TACTGAGAAA
                      20